(19) Europäisches Patentamt
European Patent Office
Office européen des brevets

(11) Veröffentlichungsnummer: **0 017 661**
**B1**

(12) # EUROPÄISCHE PATENTSCHRIFT

(45) Veröffentlichungstag der Patentschrift: **19.12.84**

(21) Anmeldenummer: **79104278.1**

(22) Anmeldetag: **02.11.79**

(51) Int. Cl.³: **G 01 N 33/72,** G 01 N 33/52, C 12 Q 1/28

(54) **Schnelldiagnostika zur Bestimmung occulten Blutes und Verfahren zu ihrer Herstellung.**

(30) Priorität: **09.12.78 DE 2853300**

(43) Veröffentlichungstag der Anmeldung:
**29.10.80 Patentblatt 80/22**

(45) Bekanntmachung des Hinweises auf die Patenterteilung:
**19.12.84 Patentblatt 84/51**

(84) Benannte Vertragsstaaten:
**CH FR GB SE**

(56) Entgegenhaltungen:
**DE-A-2 460 903**
**DE-A-2 546 252**

**N. HENNING, Klinische Laboratoriumsdiagnostik, 3. Auflage, 1966, Verlag URBAN & SCHWARZENBERG, München-Berlin-Wien, Seiten 528-529**
**Kirk-Othmer, Enzyclopedia of Chemical Technology, Third Edition, New York 1978, Vol. 1, Seite 403**

(73) Patentinhaber: **Röhm GmbH**
**Kirschenallee Postfach 4242**
**D-6100 Darmstadt 1 (DE)**

(72) Erfinder: **Krämer, Dieter, Dr.**
**An der Favorite 4**
**D-6500 Mainz (DE)**
Erfinder: **Lehmann, Klaus, Dr.**
**Schillerstrasse 85**
**D-6101 Rossdorf (DE)**
Erfinder: **Hartl, Roland**
**Forstmühlstrasse 14**
**D-6115 Münster 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 017 661 B1

**Beschreibung**

Die Erfindung betrifft Schnelldiagnostika zur diagnostischen Erfassung von Blut in Exkreten und Körperflüssigkeiten. Das Bedürfnis nach einfachen, schnellen und gleichzeitig sicheren Diagnoseverfahren hat in der Medizin unter anderem zur Entwicklung der Teststreifen-Methode geführt. Der Grundgedanke besteht darin, Reagentien auf einem festen Träger aufzubringen, die nach dem Befeuchten unter der Einwirkung der nachzuweisenden Stoffe mittelbar oder unmittelbar mit einer visuell erkennbaren Veränderung, in der Regel unter Bildung eines Farbstoffs oder unter Farbumschlag reagieren.

Große praktische Bedeutung haben die für den Nachweis okkulten Bluts in Exkreten wie Stuhl bzw. Urin und in Körperflüssigkeiten entwickelten Teststreifen erlangt. Der Test beruht auf dem Nachweis des peroxidase-wirksamen Blutbestandteils Hämoglobin. Bei Anwesenheit von Peroxid tritt unter der Einwirkung des Hämoglobins bei einer Reihe geeigneter Indikatoren (Chromogene) eine erkennbare Färbung bzw. Farbänderung auf. Als Chromogene werden einerseits Guajak-Harz, andererseits Chromogene aus der Klasse der Phenylaminoverbindungen wie z.B. o-Toluidin, m-Toluidin und ihre Säureadditionssalze, Benzidin, 3,3′, 5,5′-Tetramethylbenzidin, Diphenylamin, 4-Diphenylsulfonsäure und 2,6-Dichlorindophenol-Natrium angewendet. Eine Nachweismethode für Blut in den Faeces, unter Verwendung von Benzidin als Indikator, wird in der Literaturstelle "Klinische Laboratoriumsdiagnostik", Herausg. N. Hennig, Urban & Schwarzenberg, 1966, beschrieben. Diese sogenannte "Benzidinprobe" wird im Reagenzglas durchgeführt.

In der DE—OS 26 52 545 wird die Mitverwendung von gewissen Thiazolverbindungen als Sensibilisatoren für die Chromogene empfohlen.

Zum Nachweis von okkultem Blut im Stuhl bzw. im Urin bedarf es demnach eines geeigneten Trägers, wie z.B. ein Filterpapierstreifen, der mit der chromogenen Substanz getränkt ist (Teststreifen) und auf den eine Probe des zu untersuchenden Stuhls bzw. Urins aufgebracht wird. "Entwickelt" wird dann durch Zugabe von Peroxid-Lösung. Bei der Gestaltung des Teststreifens (Testbrief) im einzelnen sind verschiedene Lösungen vorgeschlagen worden, beispielsweise wird in der US—PS 3 996 006 ein Mehrloch-Testbrief beschrieben.

Aus der DE—A 25 46 252 sind spezifische Ausführungsformen von Teststreifen bekannt, der Testzone einen sauren Puffer vom pH 2,5—5, ein Chromogen, einen Aktivator der peroxidischen Wirkung des Hämoglobins und eine organisches Hydroperoxid in Form eines stabilen Salzes mit einem organischen Amin enthält. Die Imprägnierlösung, mit der das Hydroperoxidsalz aufgebracht wird, soll einen festen, natürlichen oder synthetischen organischen, polymeren filmbildenden Stoff enthalten. Die vorliegende Erfindung beruht auf einem anderen Prinzip der Entwicklung, wobei der Zusatz polymerer filmbildender Stoffe vom Prinzip her als nachteilig vorausgesetzt werden könnte.

In der DE—A 24 60 903 wird die Verwendung von 3,3′,5,5′-Tetraalkylbenzidin als Oxidationsindikatoren in Schnelltesten empfohlen. Die Trägerpapiere sind auch hier mit Peroxid getränkt. Die Entwicklung geschieht durch Tauchen in die zu testende Flüssigkeit.

Aus der inzwischen gewonnenen praktischen Erfahrung heraus kommt einer Forderung an die Teststreifenmethode zur Erfassung okkulten Blutes im Stuhl kritisches Gewicht zu: Die Methode soll nicht zu sensitiv sein, um falschpositive Resultate wegen ihrer pychologischen und materiellen Folgen zu vermeiden. Die Methode darf umgekehrt auch nicht zu wenig sensitiv sein, um unter allen Umständen ein falsch-negatives Resultat zu vermeiden.

In diesem Zusammenhang wirkt sich als besonders gravierende Störung aus, wenn das Chromogen bereits ohne Einwirkung des peroxidase-wirksamen Hämoglobins Farbveränderungen ähnlicher oder gleicher Art zeigt, wie die, die man in qualitativen Tests auf Hämoglobin bewertet. Derartige Störungen sind bei der Anwendung von Indikatorfarbstoffen (Chromogenen) aus der oben genannten Klasse der Phenylaminoverbindungen bekannt. Weiter beobachtet man bei Vertretern dieser Klasse von Chromogenen öfters ein unerwünscht rasches Nachlassen der Farbintensität bis zum völligen Verblassen.

Insgesamt konnten daher diese an sich leicht und in genügender Reinheit zugänglichen Indikatorfarbstoffe nur sehr bedingt als Chromogene zum Nachweis von okkultem Blut im Stuhl bzw. Urin empfohlen werden. Es wurde nun gefunden, daß Schnelldiagnostika zur diagnostischen Erfassung von Blut in Exkreten und Körperflüssigkeiten besonders geeignet sind, die aus mindestens einem festen Träger, der mit chromogenen Phenylaminoverbindungen oder Guajakharz als Indikator beladen ist bestehen, wenn das Trägermaterial mit Acryl- oder/und Methacrylharzen mit anionischem Charakter aus wäßriger Dispersion beladen ist.

Die als Chromogene verwendbaren Phenylaminoverbindungen fallen im allgemeinen unter die Formel

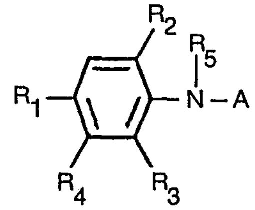

worin

$R_1$ einen hydrophilen Rest wie eine OM— oder eine $SO_3M$-Gruppe, worin

M für Wasserstoff oder ein Metallkation steht oder einen Rest

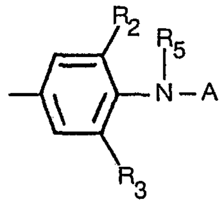

oder Wasserstoff,

$R_2$, $R_3$ und $R_4$ unabhängig voneinander Wasserstoff oder einen niederen Alkylrest, vorzugsweise einen Methylrest,

$R_5$ Wasserstoff oder zusammen mit A ein Chinonimin und

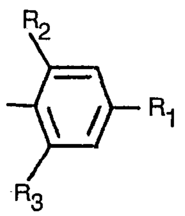

A Wasserstoff oder einen Rest bedeutet mit der Maßgabe, daß maximal zwei Phenylreste im Molekül vorhanden sind und die Summe der numerischen Werte der Indexzahlen bei $R_1$, $R_2$, $R_3$ und $R_4$ an einen Phenylrest den Wert 6 nicht überschreiten soll.

Jedoch sind auch andere Chromogene dem Verfahren gemäß der Erfindung zugänglich. Genannt seien das o- und das m-Toluidin sowie ihre Säureadditonssalze, Benzidin, 3,3',5,5'-Tetramethylbenzidin, Diphenylamin, 4-Diphenylsulfonsäure und ihre Salze und 2,6-Dichlorindophenol-Natrium.

Besonders wichtig für das erfindungsgemäße Verfahren ist das Guajak-Harz (Resina Guajaci). Es handelt sich hierbei um Harz der Guajakbäume (Guajacum officinale L. und Guajacum sanctum L.), beides in Süd- und Mittelamerika häufig vorkommende Baumarten. Über Lignum Guajaci und Gewinning des Guajakharzes in verschiedenen Qualitätsstufen informiere man sich aus 'Hagers Handbuch' der pharmazeutischen Praxis, Band I, zweiter berichtigter Neudruck, Springer Verlag (Berlin, Göttingen, Heidelberg) 1949, Seite 1397 und folgende.

Im allgemeinen ist die dort als Resina Guajaci depurata beschriebene Qualität für Anfertigung von Papierstreifen zur Testung von Blut im Stuhl oder Urin ausreichend, jedoch können durch Chromatographie aus diesen Harzsubstanzen gewonnen werden, welche die chromogene Wirkung besitzen und die zusammen oder auch jede für sich zum Imprägnieren von Testpapier geeignet sind.

In der Praxis bestimmt die Löslichkeit des Chromogens, speziell die Löslichkeit in Wasser den Applikationsmodus.

Bei wasserlöslichen Chromogenen kann die Durchführung des erfindungsgemäßen Verfahrens zweckmäßig so vorgenommen werden, daß man die wäßrige (Meth)-Acrylharzdispersion gegebenenfalls in Verdünnung zusammen mit den Chromogenen, insbesondere denen der vorstehenden Formel ansetzt, und den Träger, speziell das Filterpapier mit der so erhaltenen wäßrigen Lösung imprägniert. Wasserunlösliche Chromogene wie Guajakharz löst man in geeigneten organischen Lösungsmitteln und imprägniert damit das Papier. Anschließend kann es mit der wäßrigen Dispersions behandelt werden.*

Man kann aber das synthetische Polymere als Trockensubstanz gleichzeitig mit dem Chromogen im organischen LM lösen und beide simultan auf das Papier aufbringen.

Dabei können hinsichtlich der Auswahl der Träger, insbesondere der Filterpapiere, der Konzentration und der Auftragsweise der Farbstoff-Indikatoren die Erfahrungen des Standes der Technik übernommen werden.

Als Richtwert für die Konzentration der (Meth)-acrylharzdispersion im wäßrigen zum Imprägnieren vorgesehenen Medium kann 1 bis 10 Gew.-% vorzugsweise bis 5 Gew.-%, speziell ca. 3 Gew.-% angesehen werden. Gegebenenfalls kann die Verdünnung der ursprünglichen wäßrigen (Meth)-acrylharzdispersion, beispielsweise der Handelsformen auch unter Zusatz eines geeigneten organischen Verdünnungsmittels, wie beispielsweise Äthanol vorgenommen werden.

Entscheidend für das Gelingen des Verfahrens ist, daß mit einer ausreichenden Menge des Chromogens pro Flächen- bzw. Raumeinheit des Trägers imprägniert wird. Die pro individuelles Chromogen benötigten Mengen können weitgehend als bekannt betrachtet werden.

Als Richtwerte für den Gehalt an (Meth)-acrylharz des auf dem Filterpapier aufzubringenden Films können pro Flächeneinheit Filterpapier Werte von 0,1 bis 10 mg $Harz/cm^2$ Fläche angesehen werden.

Bei Verwendung der besonders bevorzugten Dispersionen aus Methacrylsäure/Äthylacrylat im Verhältnis 1:1 mit einem Molgewicht von ca. 250'000 kommen vorteilhaft zwischen 0,2 und 0,8 mg $Harz/cm^2$ Fläche, vorzugsweise um 0,3 mg $Harz/cm^2$ Fläche infrage.

Die Konzentration an den Chromogenen wird vorteilhafterweise so bemessen, daß zwischen 0,001—10 mg des Indikatorfarbstoffs auf einen $cm^2$ der Trägerfläche entfallen, in Abhängigkeit von den Daten des individuellen Farbstoffs. So rechnet man beispielsweise, daß zur Imprägnierung eines $cm^2$ Filterpapier eines Teststreifens 0,2 bis 200 $\mu g$ vorzugsweise 1 bis 10 $\mu g$ o-Toluidin bzw. m-Toluidin bzw. eine entsprechende Menge ihrer Säureadditionssalze

*Auch die umgekehrte Reihenfolge des jeweiligen Vorgehens ist möglich.

| | | | |
|---|---|---|---|
| 1 bis | 100 $\mu$g vorzugsweise | 10 $\mu$g | Benzidin |
| 1 bis | 200 $\mu$g vorzugsweise | 20 $\mu$g | 3,3',5,5'-Tetramethylbenzidin |
| 20 bis | 2000 $\mu$g vorzugsweise | 500 $\mu$g | Diphenylamin |
| 50 bis | 5000 $\mu$g vorzugsweise | 1000 $\mu$g | 4-Diphenylaminsulfonsäure |
| 20 bis | 1000 $\mu$g vorzugsweise | 100 $\mu$g | Guajakharz |
| 2 bis | 100 $\mu$g vorzugsweise | 10 $\mu$g | gereinigtes Guajakharz |

eingesetzt werden.

Die Imprägnierung kann in an sich bekannter Weise, beispielsweise durch Besprühen oder durch Eintauchtechniken vorgenommen werden.

Durch die Imprägnierung mit den genannten Acrylharzen läßt sich erfindungsgemäß sowohl die Empfindlichkeit als die Ablesbarkeit des Blutnachweises in Urin und Stuhl erheblich verbessern. Die Grenzen des sicheren Nachweisbereichs für Hämoglobin im Urin konnten bei Verwendung von o-Toluidiniumchlorid als Indikator von 250—1000 mg/l auf 100—5000 mg HB bei Anwendung des erfindungsgemäßen Verfahrens ausgedehnt werden.

Die Konturen der für die Erkennung maßgeblichen Farbstoffflecke sind beim erfindungsgemäßen Vorgehen schärfer und der Gesamtuntergrund zeigt weniger Eigenfärbung.

Günstig wirkt sich für das Verfahren auch das wesentlich langsamere Verblassen der Farbflecke verglichen mit den unbehandelten Proben aus. Besonders verdient hervorgehoben zu werden, daß die störenden Farbentwicklungen vor dem eigentlichen "Entwickeln" ausbleiben, wodurch die Nullwerte deutlich negativ bleiben. Man erreicht auch je nach indivudueller Empfindlichkeit eines Chromogens, seiner Menge sowie der Menge an Polymeren abgestufte Empfindlichkeiten und gewinnt vor allem eine wesentlich bessere Sicherheit bei Erkennen niedriger Blutkonzentrationen.

Erwähnt sei insbesondere die Imprägnierung mit wäßrigen Dispersionen von Harzen, die durch Copolymerisation von Methacrylsäure mit Äthylacrylat, vorzugsweise im Gewichtsverhältnis 1:1 hergestellt worden sind.

Vergleichsversuche

Die begleitende fotografische Dokumentation baut auf dem an sich bekannten Nachweis des katalytisch wirksamen Blutbestandteils Hämoglobin durch Oxidation des Indikatorfarbstoffs mit Peroxid als Oxidationsmittel auf.

A. Versuchsreihe mit o-Toluidiniumchlorid als Indikator

a. Vorbereitung der Testpapiere ohne Acrylharzimprägnierung Rundfilter ($\phi$=12,5 cm, EDEROL Nr. 1) wurden in eine wäßrige Lösung von o-Toluidiniumchlorid (0,1 g/100 ml dest. $H_2O$) getaucht und anschließend mit dem Föhn getrocknet.
(Siehe Fotografie LJ.-Nr. 5932-45e)

b. Vorbereitung der Testpapiere mit Acrylharzimprägnierung. Rundfilter, die zunächst wie unter a. vorbehandelt waren, werden in eine Dispersion eines Äthylacrylat - Methylmethacrylat - Methacrylsäure-Copolymerisats im ungefähren Verhältnis 70:30:1 (3%ig in $H_2O$) getaucht und ebenfalls mittels eines Föhns getrocknet.
(Siehe Fotografie LJ.-Nr. 5932-45f)

c. Vorbereitung wie unter b., nur daß eine andere Polymer-dispersion, nämlich aus Äthylacrylat-Methacrylsäure (im ungefähren Verhältnis 1:1,3%ig in $H_2O$ bestehend, verwendet wird.
(Siehe Fotografie LJ.-Nr. 5932-45g)

B. Versuchsreihe mit Guajak-Harz als Indikator

a'. Vorbereitung von Testpapieren ohne Acrylharzimprägnierung Rundfilter ($\phi$=12,5 cm, EDEROL Nr. 1) werden in eine Guajakharzlösung (0,5 g/100 ml Isopropanol p.a) getaucht und mit einem Föhn getrocknet.
(Siehe Fotografie LJ.-Nr. 5932-47a)

b'. Filterpapiere—wie bei a'. behandeltwerden in eine Acrylatdispersion, wie unter b. beschrieben (3%ig in $H_2O$), getaucht und mit dem Föhn getrocknet.
(Siehe Fotografie LJ.-Nr. 5932-47b).

C. Auftragung von Hämoglobin-Lösungen

Jedes gemäß A. bzw. B. imprägnierte Filterpapier wurde mit Bleistift in der unten angezeigten Weise markiert:

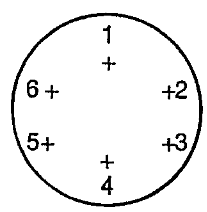


Auf die numerierten Stellen werden nun beginnend mit einem Blindwert (50 $\mu$l dest. $H_2O$) definierte Hämoglobinlösungen in ansteigender Menge aufgetragen. (Stammlösung=4% Hämoglobin mit $H_2O$ dest. auf 0,004% verdünnt.)

Auf die einzelnen numerierten Stellen wurden aufgetragen:

| | | |
|---|---|---|
| Nr. 1 | Blindwert: | 50 $\mu$l dest. $H_2O$ |
| 2 | 1 $\mu$l: | 40 ng Hämoglobin |
| 3 | 5 $\mu$l: | 200 ng Hämoglobin |
| 4 | 10 $\mu$l: | 400 ng Hämoglobin |
| 5 | 20 $\mu$l: | 800 ng Hämoglobin |
| 6 | 50 $\mu$l: | 2000 ng Hämoglobin |

D. Entwickeln

Nach dem vollständigen Aufsaugen der Hämoglobin-Lösungen wurde mit einem Tropfen Entwicklerflüssigkeit entwilkelt: (alkoholisch-wäßrige Peroxid-Lösung mit 4% Peroxidgehalt).

In Abständen von 30 sec, 2 min, 5 min und 15 min wurden Aufnahmen gemacht.

Polaroid-Kamera, Blende 16, Belichtungszeit 1/60 sec Vorsätze: Close-up-Set mit Blitzlichtfilter, Stellung M, Abstand zum Filger: 30 cm).

E. Auswertung der Ergebnisse

Beim Vergleich der den Stand der Technik repräsentierenden unbehandelten Serien Aa (Fotografie LJ.-Nr. 5932-45e) und Ba' (Fotografie LJ.-Nr. 5932-47a) mit den anmeldegemäß präparierten Serien Ab und Ac (Fotografie LJ.-Nr. 5932-45f bzw. 5932-45g) und Bb' (Fotografie LJ-Nr. 5932-47b) fällt auf Anhieb die größere Farbintensität und schärfere Begrenzung der korrespondierenden Farbflecke in den anmeldegemäßen Serien ins Auge.

Ein eingehender Vergleich zeigt, daß in den unbehandelten Serien Aa und Ba' bereits Hämoglobin gemäß Position Nr. 3, d.h. in Konzentrationen von 200 ng, nicht mehr als positiv bewertet würden, während in den anmeldegemäß präparierten Serien Ab und Bb' kein Zweifel an dem positiven Resultat bestehen kann. Bei den anmeldegemäß präparierten Serien gibt für den Fachmann bis zum Ablauf von 2 min sogar die niedrigste Kondentration (Position 2) noch ein positives Resultat.

Bei den Filtern Aa und Ba', die den Stand der Technik repräsentieren, sind ab 2 Minuten nach Entwicklung eindeutig positive Resultate überhaupt nicht mehr zu erhalten. Ganz anders die Situation bei den anmeldegemäß präparierten Ab, Ac und Bb'.

Zusammenfassend stellen wir fest, daß durch die beiliegende fotographische Dokumentation die Überlegenheit der anmeldegemäßen Ausführung der infragestehenden Tests tatsächlich ad oculos demonstriert wird.

**Patentansprüche**

1. Schnelldiagnostika zur diagnostischen Erfassung von Blut in Exkreten und Körperflüssigkeiten, bestehend aus mindestens einem festen Träger, der mit chromogenen Phenylaminoverbindungen oder Guajakharz, als Indikator beladen ist, dadurch gekennzeichnet, daß das Trägermaterial mit Acryl- oder/und Methacrylharzen mit anionischem Charakter aus wäßriger Dispersion imprägniert ist.

2. Verfahren zur Herstellung von Schnelldiagnostika nach Anspruch 1, dadurch gekennzeichnet, daß man mit wäßrigen Dispersionen von Harzen imprägniert, die durch Copolymerisation von Methacrylsäure mit Äthylacrylat, vorzugsweise im Gewichtsverhältnis 1:1, hergestellt worden sind.

3. Verfahren zur Herstellung von Schnelldiagnostika nach Anspruch 1, dadurch gekennzeichnet, daß man mit einer wäßrigen Dispersion eines Äthylacrylat-Methylmethacrylat-Methacrylsäurecopolymerisats im ungefähren Gewichtsverhältnis 70:30:1 imprägniert und daß mannals Chromogene Phenylaminoverbindung o-Toluidiniumchlorid verwendet.

**Revendications**

1. Matériels de diagnostic rapide pour la détection, à des fins de diagnostic, de la présence de sang dans les excréments et les liquides biologiques, se composant d'au moins un support solide qui est chargé de composés phénylaminés chromogènes ou de résine de gaïac servant d'indicateur, caractérisés en ce que la matière de support est imprégnée par des résines acryliques et/ou méthacryliques de caractère anionique à partir d'une dispersion aqueuse.

2. Procédé pour la fabrication de matériels de diagnostic rapide selon la revendication 1, caractérisé en ce qu'on procède à l'imprégnation avec des dispersions aqueuses de résines qui ont été préparées par copolymérisation d'acide méthacrylique avec l'acrylate d'éthyle, de préférence dans le rapport pondéral 1:1.

3. Procédé pour la fabrication de matériels de diagnostic rapide selon la revendication 1, caractérisé en ce qu'on procède à l'imprégnation avec une dispersion aqueuse d'un copolymère d'acrylate d'éthyle-méthacrylate de méthyle-acide méthacrylique dans le rapport pondéral approximatif 70:30:1, et en ce qu'on utilise, comme composé phénylaminé chromogène, le chlorure d'o-toluidine.

**Claims**

1. Rapid diagnostic agents for diagnostically detecting blood in excreta and body fluids, consisting of at least one solid carrier which is charged with chromogenic phenylamino compounds or guaiacum resin as indicator, characterised in that the carrier material is impregnated with acrylic and/or methacrylic resins of an anionic nature from an aqueous dispersion.

2. Process for producing rapid diagnostic agents as claimed in claim 1, characterised in that impregnation is carried out with aqueous dispersions of resins which have been prepared by copolymerisation of methacrylic acid with ethyl acrylate, preferably in the weight ratio 1:1.

3. Process for producing rapid diagnostic agents as claimed in claim 1, characterised in that the impregnation is carried out with an aqueous dispersion of an ethyl acrylate/methyl acrylate/methacrylic acid copolymer in a weight ratio of approximately 70:30:1 and that o-toloidinium chloride is used as a chromogenic phenylamino compound.

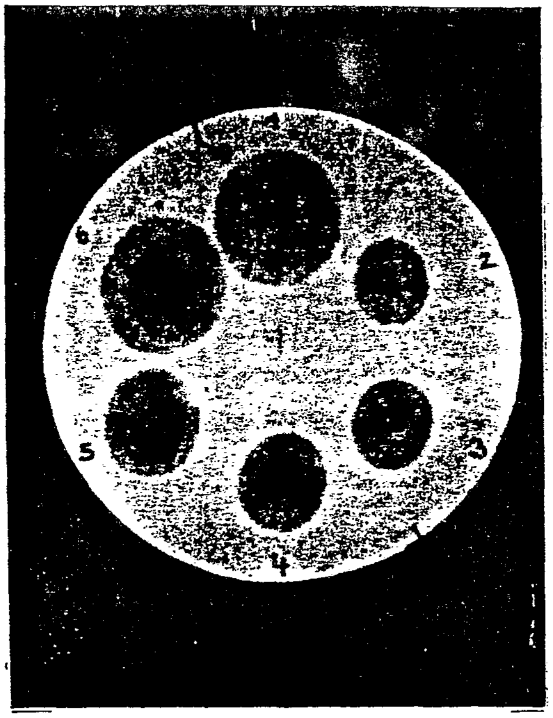

Nr. 1 Ederol 1 o-TDCl 0,1 %

30 sec.
LJ.Nr. 5932-45 e

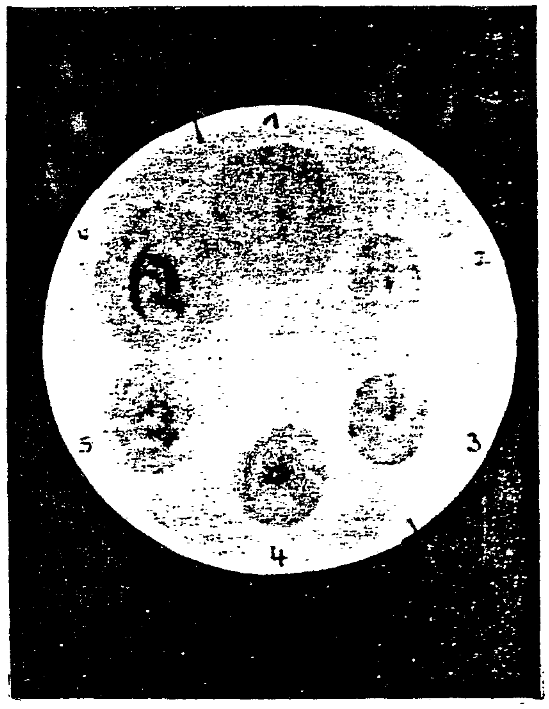

Nr. 2 Ederol 1 o-TDCl 0,1 %

2 min.
LJ.Nr. 5932-45 e

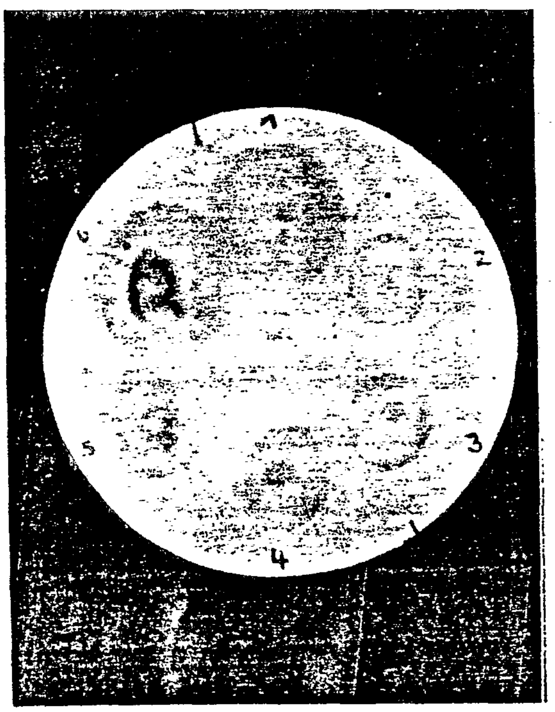

Nr. 3 Ederol 1 o-TDCl 0,1 %

5 min.
LJ.Nr. 5932-45 e

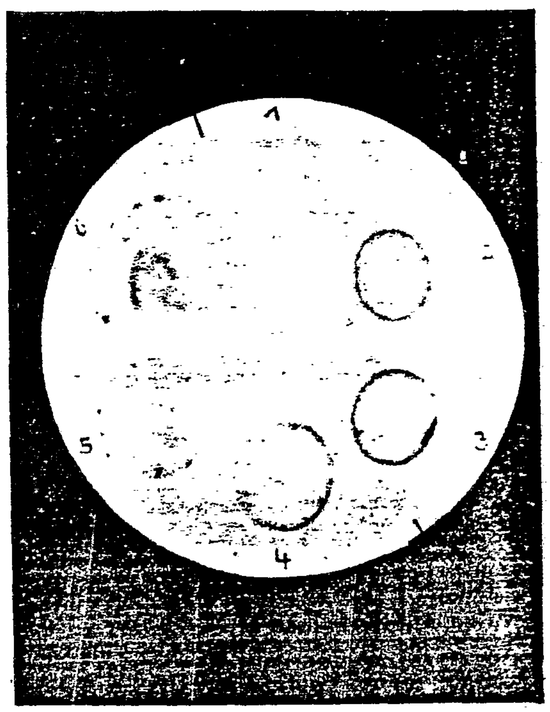

Nr. 4 Ederol 1 o-TDCl 0,1 %

15 min.
LJ.Nr. 5932-45 e

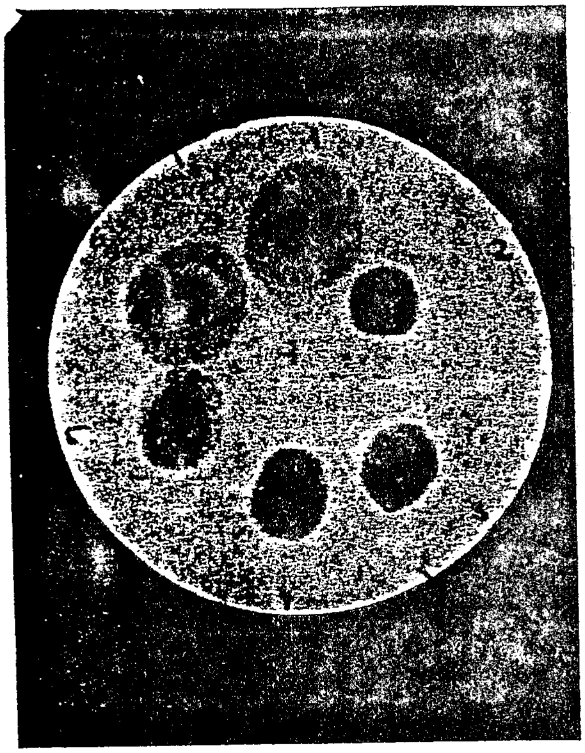

Nr. 5 Ederol 1 o-TDCl 0,1 %
E 30 D Dispersion 3 %
30 sec
LJ.Nr. 5932-45 f

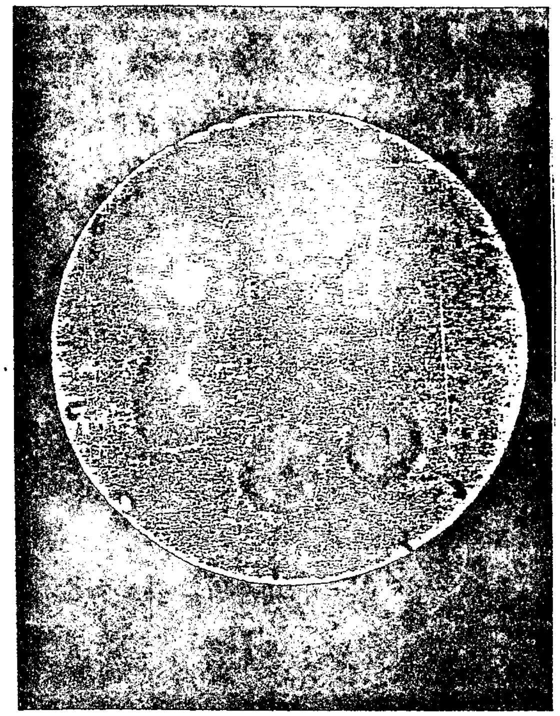

Nr. 6 Ederol 1 o-TDCl 0,1 %
E 30 D Dispersion 3 %
2 min.
LJ.Nr. 5932-45 f

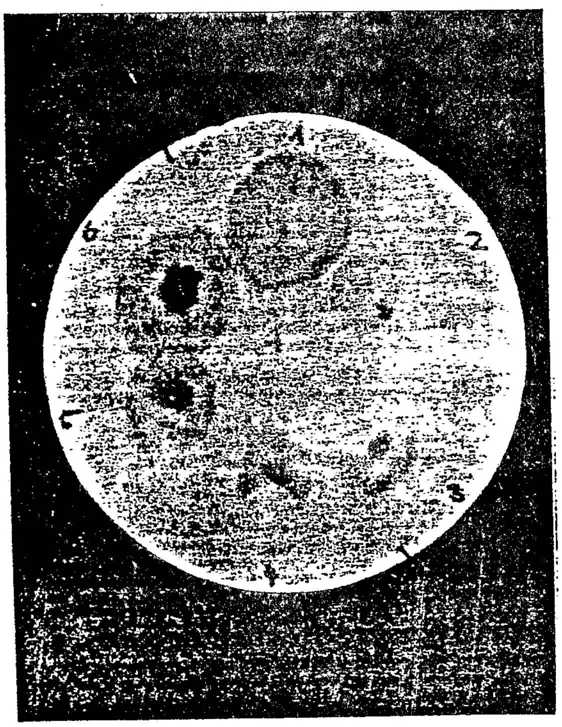

Nr. 7 Ederol 1 o-TDCl 0,1 %
E 30 D Dispersion 3 %
5 min.
LJ.Nr. 5932-45 f

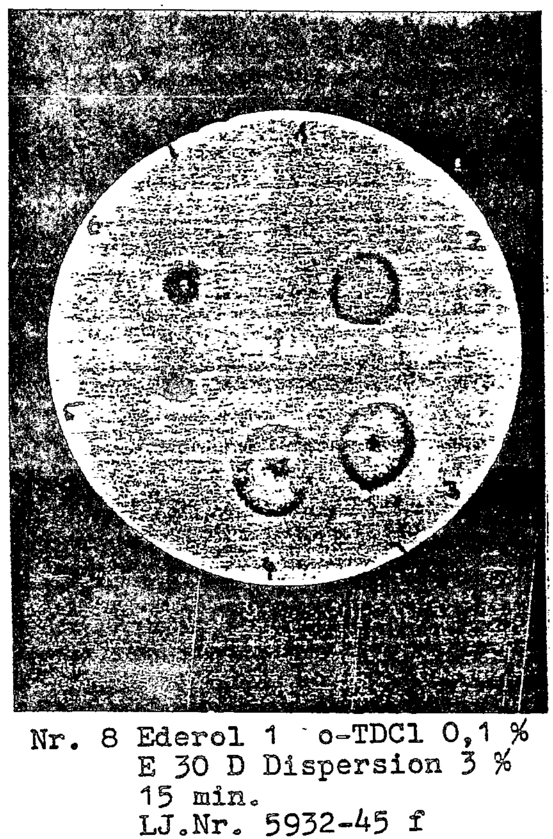

Nr. 8 Ederol 1 o-TDCl 0,1 %
E 30 D Dispersion 3 %
15 min.
LJ.Nr. 5932-45 f

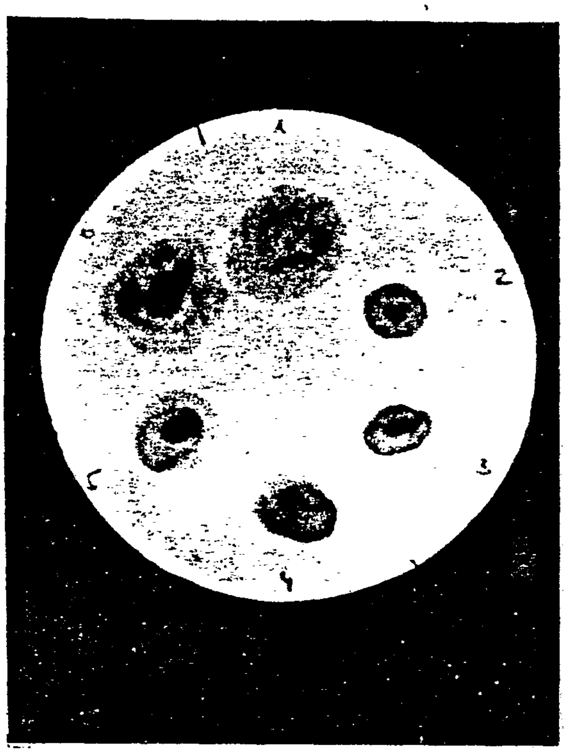

Nr. 9 Ederol 1 o-TDCl 0,1 %
L 30 D Dispersion 3 %
30 sec.
LJ.Nr. 5932-45 g

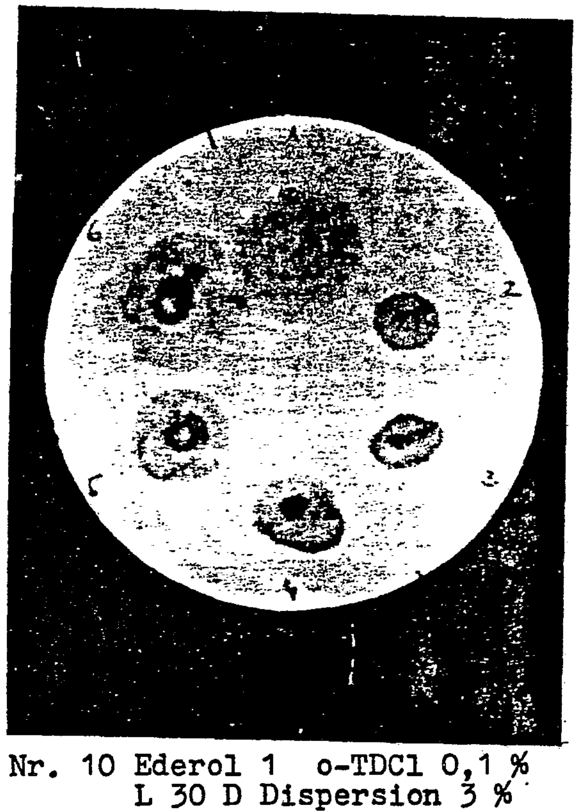

Nr. 10 Ederol 1 o-TDCl 0,1 %
L 30 D Dispersion 3 %
2 min.
LJ.Nr. 5932-45 g

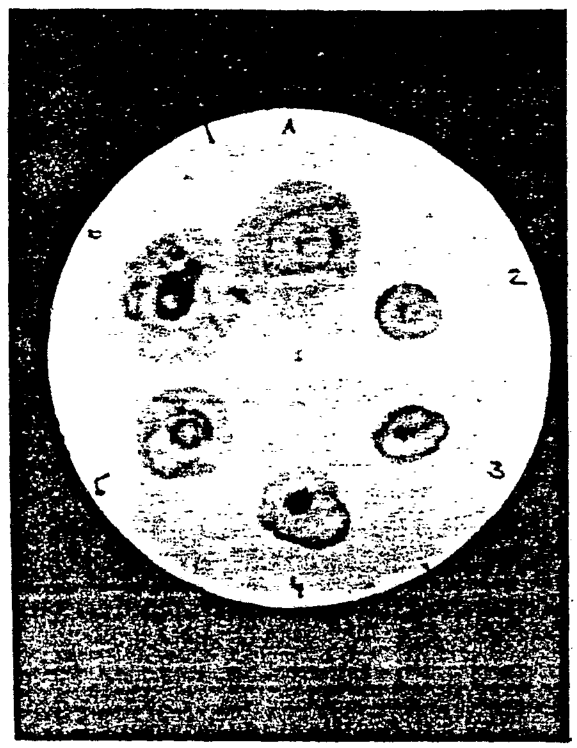

Nr. 11 Ederol 1 o-TDCl 0,1 %
L 30 D Dispersion 3 %
5 min.
LJ.Nr. 5932-45 g

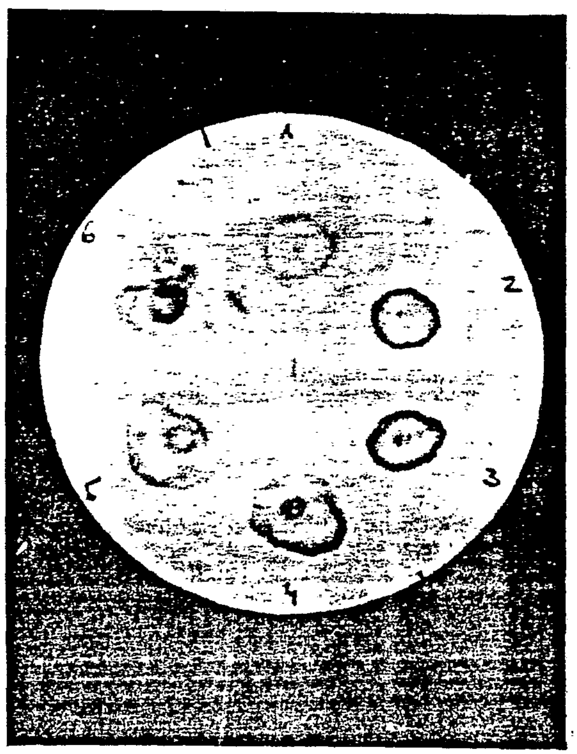

Nr. 12 Ederol 1 o-TDCl 0,1 %
L 30 D Dispersion 3 %
15 min.
LJ.Nr. 5932-45 g

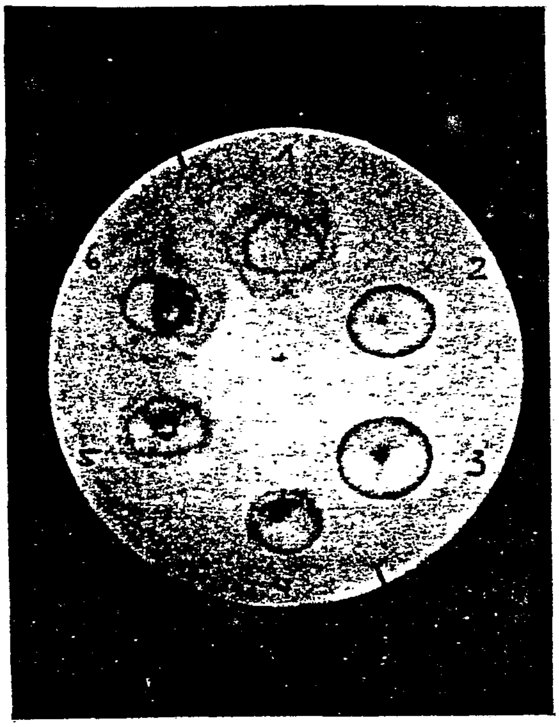


Nr. 13 Ederol 1 Guajakharz 0,5%

30 sec.
LJ.Nr. 5932-47 a

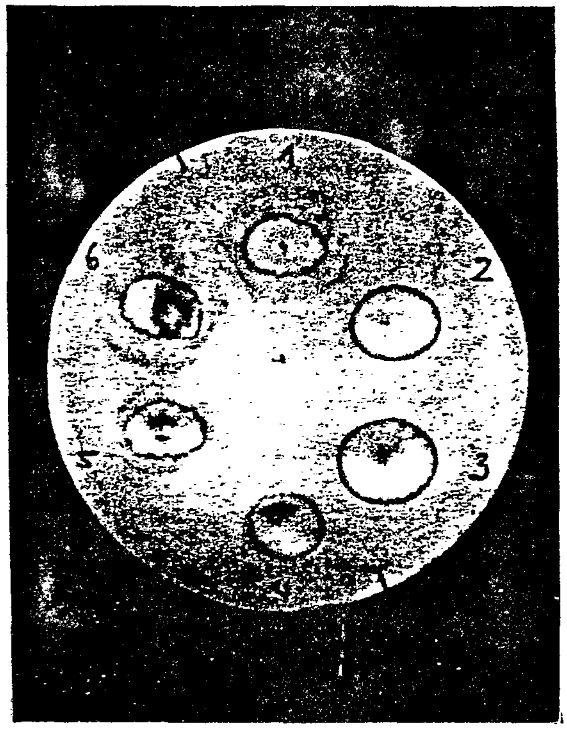


Nr. 14 Ederol 1 Guajakharz 0,5%

2 min.
LJ.Nr. 5932-47 a

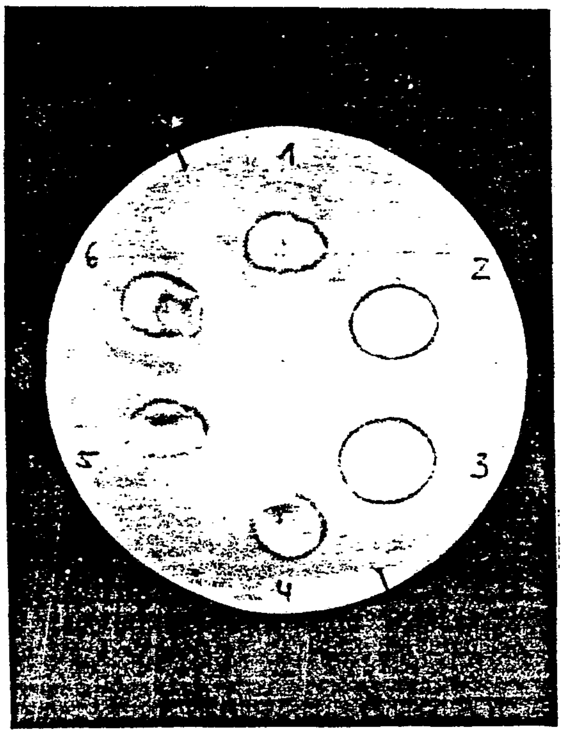


Nr. 15 Ederol 1 Guajakharz 0,5%

5 min.
LJ.Nr. 5932-47 a

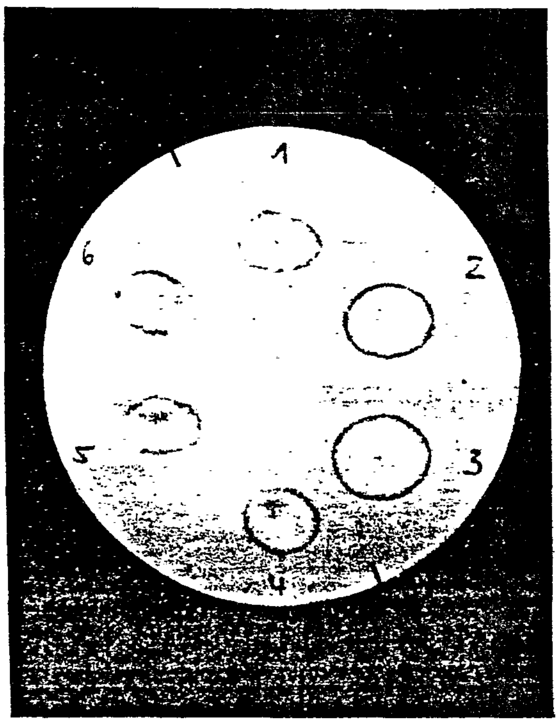


Nr. 16 Ederol 1 Guajakharz 0,5%

15 min.
LJ.Nr. 5932-47 a

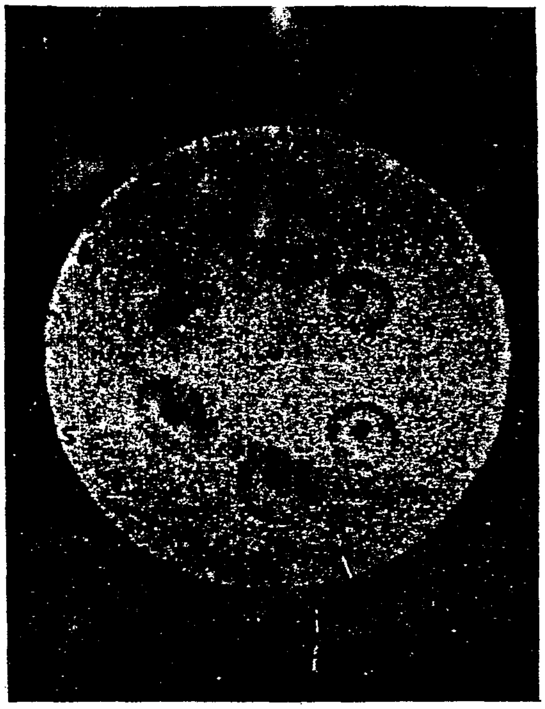

Nr. 17 Ederol 1 Guajakharz 0,5%
E 30 D Dispersion 3 %
30 sec.
LJ.Nr. 5932-47 b

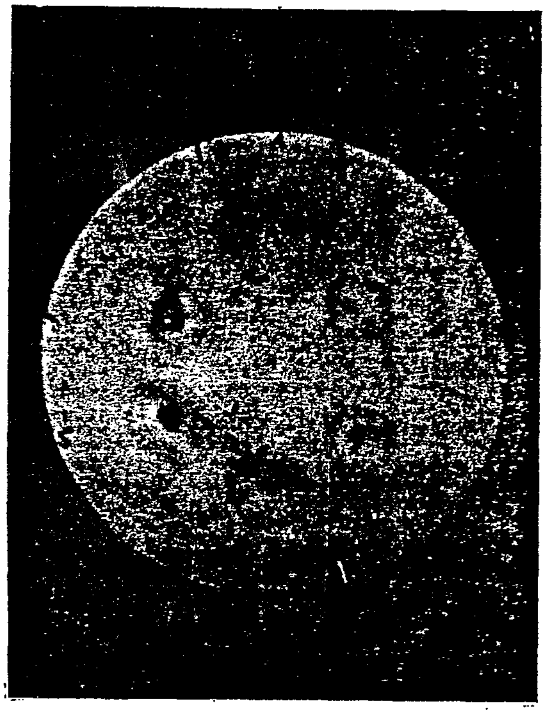

Nr. 18 Ederol 1 Guajakharz 0,5%
E 30 D Dispersion 3 %
2 min.
LJ.Nr. 5932-47 b

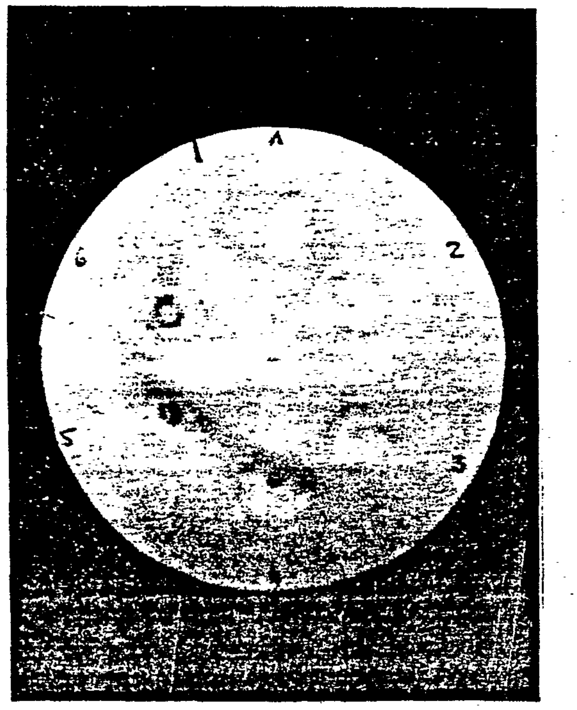

Nr. 19 Ederol 1 Guajakharz 0,5%
E 30 D Dispersion 3 %
5 min.
LJ.Nr. 5932-47 b

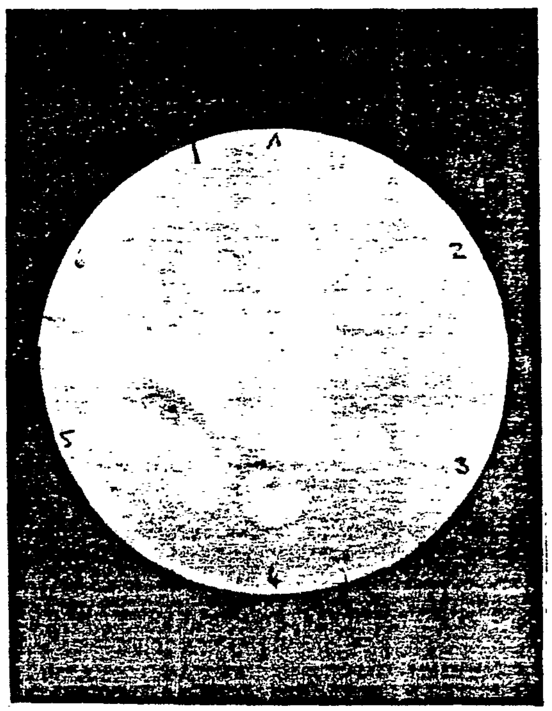

Nr. 20 Ederol 1 Guajakharz 0,5%
E 30 D Dispersion 3 %
15 min.
LJ.Nr. 5932-47 b